# FASCICULE DE BREVET EUROPEEN

(11) **EP 2 632 883 B1**
(45) Date de publication et mention de la délivrance du brevet: **27.09.2017**
(21) Numéro de dépôt: 11787736.5
(22) Date de dépôt: 26.10.2011
(51) Int. Cl.: C07C 45/52, C07C 47/22, C07C 319/18, C07C 319/20, C07C 323/22, C07C 323/52, C07C 323/58, C07C 323/60

(54) **PROCÉDÉ D'OBTENTION D'ACROLÉINE PAR DÉSHYDRATATION CATALYTIQUE DE GLYCÉROL OU DE GLYCÉRINE**
VERFAHREN ZUR GEWINNUNG VON ACROLEIN MITTELS KATALYSATORDEHYDRIERUNG VON GLYCEROL ODER GLYCERIN
PROCESS FOR OBTAINING ACROLEIN BY CATALYTIC DEHYDRATION OF GLYCEROL OR GLYCERIN

(30) Priorité: 26.10.2010 FR 1058767
(43) Date de publication de la demande: 04.09.2013
(73) Titulaire: Adisseo France S.A.S., 92160 Antony (FR); Ecole Centrale de Lille, 59651 Villeneuve D' Ascq (FR)
(72) Inventeur: PARIENTE, Stéphane, F-93170 Bagnolet (FR); BELLIERE-BACA, Virginie, F-78570 Andresy (FR); PAUL, Sébastien, F-59158 Thun Saint-Amand (FR); FATAH, Nouria, F-59175 Vendeville (FR)
(74) Mandataire: Delorme, Nicolas
(86) Numéro de dépôt international: PCT/FR2011/052492
(87) Numéro de publication internationale: WO 2012/056166

(56) Documents cités:
- WO-A2-2006/087083
- FR-A1- 2 920 767
- FR-A1- 2 938 535
- HERGUIDO J ET AL: "On the use of fluidized bed catalytic reactors where reduction and oxidation zones are present simultaneously", CATALYSIS TODAY, ELSEVIER, NL, vol. 100, no. 1-2, 15 February 2005 (2005-02-15), pages 181-189, XP027834496, ISSN: 0920-5861 [retrieved on 2005-02-15]

## Description

La présente invention concerne la production catalytique d'acroléine par déshydratation de glycérol ou de glycérine. Plus précisément, l'invention concerne un procédé continu d'obtention d'acroléine à partir de glycérol ou de glycérine, en présence d'un catalyseur acide, dans un réacteur catalytique à lit fluidisé, permettant de lever les insuffisances des procédés connus liées au cokage des catalyseurs hétérogènes employés.

L'acroléine, de formule H₂C=CH-CHO, est le plus simple des aldéhydes insaturés. Elle constitue un intermédiaire important de l'industrie chimique et agroalimentaire. Elle est en effet un précurseur de l'acide acrylique, qui joue un rôle important dans la fabrication des matières plastiques et des peintures. Cependant, l'utilisation la plus importante de l'acroléine est la synthèse de la D,L-méthionine, laquelle est un acide aminé essentiel pour l'alimentation animale et qui ne possède pas, ou très peu, de sources naturelles. Généralement, l'acroléine est issue de l'oxydation sélective du propylène par l'oxygène de l'air sur des catalyseurs complexes d'oxydes mixtes de bismuth et de molybdène (voir par exemple, G. W. Keulks, L. D. Krenzke, T. M. Notermann, Adv. Catal. 1978, 27, 183). La sélectivité en acroléine obtenue par ce procédé est supérieure à 80% à forte conversion du propylène (supérieure à 90%). Ce dernier étant issu du craquage catalytique des coupes pétrolières, sa disponibilité et son prix sont donc dépendants de ceux des ressources fossiles.

Dans ce contexte, l'obtention d'acroléine à partir d'une matière première renouvelable par le biais d'un procédé industriel performant et stable dans le temps présente un fort intérêt. C'est pourquoi de nombreuses études ont été menées sur la synthèse de l'acroléine par déshydratation du glycérol (voir par exemple la revue de B. Katryniok, S. Paul, M. Capron et F. Dumeignil, ChemSusChem 2009, 2, 719 - 730). En effet, le glycérol est obtenu à raison de 100 kg par tonne de biodiesel produit par transestérification des huiles végétales. La directive européenne 2003/03/30EC ayant fixé la part de marché du biodiesel dans les carburants à 10 % pour 2015, la production de ce dernier a connu une très forte croissance ces dernières années (5,7 millions de tonnes produites en Europe en 2007, chiffre qui devrait doubler d'ici 2012). Cet accroissement de production s'accompagne bien entendu mécaniquement d'une augmentation très significative de la quantité de glycérol disponible sur le marché.

Quelques exemples d'études concernant la réaction de déshydratation du glycérol en acroléine sont présentés ci-dessous afin d'illustrer les verrous technologiques que la présente invention permet de lever et ainsi dégager les avancées obtenues.

Il est connu depuis longtemps qu'une catalyse acide permet de réaliser la déshydratation du glycérol en acroléine. En effet, H. Adkins et W. H. Hartung, Organic Synthesis I, 15-18 (1964) ont montré cette possibilité à 190-200 °C grâce à un traitement du glycérol sur des poudres de sulfate de potassium et d'hydrogénosulfate de potassium. Le document US2558520A relate la déshydratation du glycérol sur des terres diatomées imprégnées avec de l'acide ortho-phosphorique pour donner un rendement en acroléine de 72,3 % à 283 °C. Dans ces travaux, la température de réaction est élevée, ce qui est néfaste à l'obtention d'une bonne sélectivité en acroléine.

Dans le document US5387720A, la déshydratation est conduite en phase gazeuse ou liquide dans un réacteur de type lit fixe. Les catalyseurs utilisés sont des solides acides avec des acidités de Hammett inférieures à +2, et de préférence inférieures à -3. La réaction en phase gazeuse donne des conversions en glycérol proche de 100 % à des températures comprises entre 250 et 340 °C, avec un rendement en acroléine de 70,5 % et d'environ 10 % en hydroxypropanone sous flux réactionnel de 40 mL.min⁻¹ d'une solution aqueuse de glycérol à 20 % en poids durant une période de 60 h. D'autres sous-produits sont également détectés lors de la réaction de déshydratation du glycérol en acroléine tels que le propanaldéhyde, l'acétaldéhyde, l'acétone ainsi que d'autres composés qui conduisent à la formation de coke sur le catalyseur et donc à sa désactivation rapide. La durée de vie du catalyseur est donc très faible dans ce cas.

Les documents WO2007/058221A1 et JP2008-088149A décrivent l'utilisation d'hétéropolyacides greffés sur silice comme catalyseur de déshydratation du glycérol en acroléine. La réaction est menée en réacteur à lit fixe avec une solution aqueuse de glycérol à 10 % en poids et à des températures comprises entre 250 et 325 °C. Le temps de réaction est de 5 h. Une comparaison est effectuée entre les catalyseurs hétéropolyacides supportés et différents types de catalyseurs acides tels que Al₂O₃, SiO₂-Al₂O₃, SiO₂, TiO₂, ZrO₂.... Les conversions de glycérol sont toujours proches de 100 % et les meilleurs rendements en acroléine atteignent 87 %. La réaction n'est pas menée pour des temps de réaction plus long que 5 h à cause de la forte désactivation subie pour une période de travail du catalyseur plus longue.

Le document WO2009/029540A2 concerne l'utilisation de différents supports solides tels que Al₂O₃, ZrO₂, SiO₂-AlO₃, SiO₂-Al₂O₃, l'Alundum, SiO₂ ou le Ludox AS30 sur lesquels sont imprégnés des phosphates métalliques, de formule M_{0,33}H_{2,33}PO₄, avec M = Ba, Cr, Mn, Fe, Co, Ni, Zn, La, Ru, ou Mo. Des catalyseurs à base de Nb, d'acide tungstique, et d'acide phosphomolybdique sont également décrits dans ce document. La réaction est menée en lit fixe avec des solutions aqueuses de glycérol à des teneurs comprises entre 17 % et 30 % en poids et à des températures allant de 250 à 320 °C. Les rendements en acroléine atteignent 87 % mais ni le temps de réaction, ni les données concernant une quelconque régénération du catalyseur ne sont fournis, sachant qu'à ces températures et avec ces rendements, la production de coke sur le catalyseur est inévitablement importante.

Pour que ces résultats, obtenus avec quel que catalyseur que ce soit, puissent être suffisamment probants sur le plan économique et ainsi déboucher sur un véritable procédé industriel, il faut donc résoudre le problème de dépôt de coke sur le catalyseur en limitant sa formation et/ou en l'éliminant périodiquement ou en continu.

Le document JP2008-110298A aborde le sujet de la régénération du catalyseur en réacteur à lit fixe avec un catalyseur de type zéolithique. Des cycles de 12 h de réaction suivis de 18 h de régénération sous air sont menés afin de récupérer l'activité initiale. On constate ici que le temps de régénération est 1,5 fois supérieur à la durée de travail du catalyseur, ce qui représente un frein majeur en terme de productivité.

Le document DE102008038273A1 traite de la mise en oeuvre de la déshydratation du glycérol en phase gazeuse dans deux réacteurs à lit fixe placés en parallèle. Le procédé prévoit en effet l'utilisation d'un premier réacteur jusqu'à une désactivation équivalente à une perte d'activité de 10 % puis un changement de réacteur afin de régénérer en parallèle le catalyseur coké sans arrêter la production. Ce procédé efficace est coûteux puisqu'il nécessite l'utilisation de deux réacteurs en parallèle.

La société Arkema a déposé de nombreuses demandes de brevet relativement à des procédés catalytiques de déshydratation du glycérol en acroléine couplés le plus souvent à une seconde étape d'oxydation de l'acroléine obtenue en acide acrylique. Le document FR2938535A1 décrit un procédé de préparation de l'aldéhyde méthylthiopropionique, comprenant une étape d'obtention d'acroléine à partir de glycérol en phase liquide ou gazeuse, dans un réacteur à lit fluidisé contenant un catalyseur. Notamment, dans le document FR2882052A1, il est décrit que l'ajout d'oxygène moléculaire dans le mélange gazeux alimentant un réacteur à lit fixe permet de prévenir/limiter la formation de coke et de composés aromatiques tels que le phénol ainsi que d'autres sous-produits provenant d'une hydrogénation de produits déshydratés comme le propanaldéhyde et l'acétone, mais aussi l'hydroxypropanone. Dans ces conditions, il est ainsi indiqué, dans le document WO2009/127889A1, des rendements en acroléine par déshydratation du glycérol de l'ordre de 93% pour des températures de réaction allant de 260 à 350 °C sur des catalyseurs de type sels d'hétéropolyacides siliciques ou phosphoriques dopés avec divers éléments tels que Cs, Rb, Ca, Fe, Zr, La, Hf ou Bi. La réaction est menée en lit fixe avec un pourcentage inférieur à 7 % en oxygène moléculaire dans l'alimentation afin de rester en dehors des limites d'explosivité des mélanges acroléine-air. Aucune donnée contenue dans ce document ne permet de conclure quand à la stabilité dans le temps de tels systèmes. Toutefois, une publication (A. Alhanash, E. F. Kozhevnikova, I. V. Kozhevnikov, Applied Catalysis A: General 378 (2010) 11-18) fait état de l'utilisation de catalyseurs identiques à ceux testés dans le document WO2009/127889A1 et une rapide perte de rendement en acroléine en fonction du temps de réaction est alors observée. En effet, le rendement en acroléine passe de 98 % après une heure sous flux réactionnel à seulement 40 % environ après 6 h sous flux. Il est donc fort probable que la tendance soit la même pour le procédé, objet du document WO2009/127889A1.

Le document FR2920767A1 décrit un procédé de préparation d'acroléine à partir de glycérol en phase gazeuse, dans un premier réacteur à lit fluidisé contenant un catalyseur acide. Le catalyseur est soutiré du lit fluidisé puis transféré dans un second réacteur à lit fluidisé où il est régénéré.

Le document WO2008/052993A2 traite de la production d'acroléine à partir de glycérol dans un lit circulant constitué principalement de zéolithe ZSM-5 et de billes d'argile. A des températures comprises entre 290 et 500 °C, avec des solutions aqueuses de glycérol à 20 %, 50 % ou 85 % en poids, la conversion du glycérol est toujours proche de 100 % mais la sélectivité en acroléine reste faible, c'est-à-dire, autour de 60 %. En outre, l'utilisation d'un réacteur à lit circulant présente des désavantages non négligeables pour une application industrielle, tels que la grande quantité de catalyseur à mettre en oeuvre, la difficulté de conduire une telle unité ou encore la nécessité de disposer d'un catalyseur suffisamment résistant aux contraintes mécaniques extrêmement fortes rencontrées dans ce type de réacteur (phénomène d'attrition).

Tous les procédés décrits dans les précédentes références pour effectuer la déshydratation catalytique du glycérol en acroléine présentent des limitations importantes en terme de durée de vie des catalyseurs, ce qui est rédhibitoire à une viabilité industrielle. Les techniques divulguées pour augmenter la durée de vie de ces catalyseurs (décokage en continu) engendrent soit des pertes de productivité en acroléine importantes, soit des coûts d'investissement très importants. Des verrous similaires sont rencontrés dans d'autres domaines d'applications et des solutions intéressantes ont été proposées.

Le document US3669877A publié en 1972, décrit la réaction de déshydrogénation du butane sur catalyseur chrome-aluminium dans un réacteur à lit fluidisé dont la partie basse au-dessus du distributeur est un simple lit fluidisé et dont la partie haute est divisée en deux zones annulaires grâce à l'ajout d'un cylindre creux dans le réacteur. La zone ainsi créée à l'intérieur du cylindre creux dans la partie haute sera la zone de réduction. La partie annulaire autour de ce cylindre sert de zone de régénération (oxydation). Enfin, la partie basse permet au solide de circuler entre les deux zones hautes. L'alimentation dans la partie cylindrique creuse se fait avec un gaz réducteur et dans la partie annulaire haute avec un gaz oxydant tel que l'oxygène permettant la régénération du catalyseur.

Le document US4152393A publié en 1979, relate l'invention d'un réacteur à lit entrainé, n'utilisant pas la technologie des lits fluidisés. Ce réacteur est divisé en quatre compartiments verticaux où le solide circule de bas en haut ou de haut en bas : le coeur du réacteur, qui sert de zone de régénération, est entouré de trois anneaux concentriques successifs. Le solide est entrainé par un gaz à partir du coeur du réacteur suivant un mouvement ascendant vers le premier anneau (le plus proche du coeur du réacteur), où les particules se déplacent suivant un courant descendant, cet anneau servant à séparer les gaz de réaction et de régénération. Par la suite, le solide est entrainé dans le deuxième anneau (mouvement du solide ascendant), qui constitue la partie réactive. Enfin, le troisième anneau sert à faire recirculer le solide de la partie réactive vers le coeur du réacteur.

Le document US6197265B1 fait également état d'un réacteur à lit fluidisé cette fois à deux zones, celles-ci étant créées par un système original de distribution du gaz au sein du lit fluidisé. Il s'agit ici de séparer une zone d'oxydation et une zone de réduction.

Un réacteur de type lit fluidisé possédant deux zones dans lesquelles la densité du solide est différente grâce à des débits de gaz différents, avec une section de réacteur plus importante dans la partie la plus dense dans le but d'éviter l'entrainement des particules, est décrit dans le document US2007/0213573A1. Ce réacteur est utilisé pour des réactions de craquage catalytique.

Un procédé de préparation d'un composé vinylique aromatique tel que le styrène, à partir d'un alkyl aromatique tel que l'éthylbenzène par déshydrogénation de ce dernier dans un réacteur de type lit fluidisé à deux zones (une zone de déshydrogénation et une zone de régénération) fait l'objet du document WO0144146A1. Ce document fait également état d'un système d'injection immergé à différentes hauteurs dans le lit fluidisé afin d'effectuer la séparation des deux zones. Vu qu'il s'agit ici de réactions de déshydrogénation, le catalyseur va subir une phase de cokage dans la partie haute du réacteur et le coke sera brûlé dans la partie basse, ce qui permet d'obtenir un procédé présentant une conversion et une sélectivité stable sur une période de plus de 200 h.

Enfin, plus récemment, le document WO2009/153382A1 décrit un lit fluidisé à deux zones avec injection du réactif dans une partie haute conique du réacteur pour des réactions d'oxydation, d'oxydation déshydrogénante et de déshydrogénation. Cette configuration est susceptible d'apporter une séparation plus nette entre les deux zones.

Les auteurs de la présente invention proposent donc de s'appuyer sur une technologie de réacteur à lit fluidisé possédant une zone de réaction et une zone de régénération pour développer un procédé performant et stable pour la production d'acroléine à partir de glycérol ou de glycérine. Contrairement à l'art antérieur exposé ci-dessus, l'invention porte sur une utilisation inédite de ce type de réacteur pour une réaction de déshydratation.

Ainsi, l'invention décrite dans le présent brevet propose d'effectuer la déshydratation du glycérol en acroléine dans un réacteur catalytique à lit fluidisé comprenant une zone réactive et une zone régénérative dans la même enceinte ce qui permet le décokage - et donc la régénération - en continu du catalyseur mis en oeuvre au sein du réacteur. Cette invention permet donc de produire en continu de l'acroléine à partir de glycérol ou de glycérine, tout en conservant les performances élevées dans le temps des catalyseurs acides utilisés.

Ainsi l'invention a pour objet un procédé continu d'obtention d'acroléine par déshydratation catalytique de glycérol ou de glycérine, en présence d'un catalyseur acide, ledit procédé comprenant la régénération concomitante dudit catalyseur et étant réalisé dans un réacteur à lit fluidisé, ledit réacteur comprenant deux zones, une première zone, ou zone inférieure, dite de régénération du catalyseur dans laquelle un gaz de fluidisation comprenant de l'oxygène est introduit et une seconde zone, ou zone supérieure, dite de réaction dans lequel le glycérol ou la glycérine est introduit et converti en acroléine.

Avant d'aborder plus en détails l'invention, les termes « glycérol » et « glycérine », sont définis. Selon l'invention, on entend par glycérol, un glycérol purifié ou non, de préférence issu de la biomasse, et notamment un glycérol hautement purifié ou partiellement purifié. Un glycérol purifié possède une pureté supérieure ou égale à 98%, obtenue par distillation de glycérine. On entend par glycérine, notamment, une glycérine d'origine naturelle, issue de l'hydrolyse d'huiles végétales et de graisses animales, ou une glycérine d'origine synthétique, issue du pétrole, plus ou moins purifiée ou raffinée, ou bien brute. Ainsi, dans la suite de la description, la référence au glycérol ou à la glycérine s'étend à tout glycérol et glycérine, quels que soient leur origine, notamment naturelle ou synthétique, et leur degré de pureté.

Comme indiqué précédemment, le réacteur comporte deux zones, supérieure et inférieure. Avantageusement, il présente la structure suivante :
la zone supérieure du réacteur comprend, de bas en haut, i) une partie d'introduction du glycérol ou de la glycérine, ii) une partie de réaction par déshydratation catalytique du glycérol ou de la glycérine, et iii) une partie de désengagement des particules solides fines formées, et/ou
la zone inférieure du réacteur comprend, de bas en haut, une partie conique d'introduction du gaz de fluidisation et une zone de régénération du catalyseur par le gaz de fluidisation.

Un résultat optimal est obtenu lorsque la mise en oeuvre du procédé ci-dessus répond aux caractéristiques préférentielles ci-après, considérées seules ou en combinaison :
le glycérol ou la glycérine est introduit sous la forme d'une solution aqueuse en une concentration variant de 10 à 90% en poids; avantageusement, ladite solution est introduite sous forme vaporisée ; l'injection du mélange réactionnel, c'est-à-dire la solution de glycérol ou de glycérine, peut être effectuée à différentes hauteurs dans le lit ;
la température de déshydratation varie de 180 à 500°C ;
le gaz de fluidisation est choisi parmi l'air, O₂ et un mélange O₂-N₂ contenant jusqu'à 21 % molaire de dioxygène ; le pourcentage d'oxygène présent dans la partie basse du lit fluidisé peut être ajusté afin de brûler en continu le coke formé dans la partie haute du lit ;
le gaz de fluidisation est chauffé à une température variant de 180 à 800°C, de préférence à une pression variant de la pression atmosphérique à 10 bars.

Un autre avantage majeur de cette invention réside dans l'utilisation possible d'une large gamme de catalyseurs acides solides fluidisables, pour la production d'acroléine à partir de glycérol ou de glycérine, les conditions opératoires dans le réacteur pouvant être ajustées aux conditions de fonctionnement du catalyseur acide utilisé. Ainsi, à titre d'exemples non limitatifs, le catalyseur acide peut être choisi parmi les zéolithes, les phosphates (tels que les phosphates de fer), les hétéropolyacides, éventuellement supportés et/ou dopés, les catalyseurs de types oxydes, oxydes supportés, ou encore de type zircones modifiées et/ou dopées, ainsi que tout autre type de catalyseur acide adapté à la déshydratation du glycérol en acroléine et présentant une résistance à l'attrition suffisante pour être employé en lit fluidisé. Selon une des variantes de l'invention, le catalyseur est choisi parmi les hétéropolyacides supportés ou non, dopés par au moins un métal choisi parmi Cs, Rb, Ca, Fe, Zr, La, Hf et Bi.

La présente invention permet de produire en continu de l'acroléine à partir de glycérol ou de glycérine sur de très longues durées. Les propriétés particulières des lits fluidisés permettent de conserver des compositions très partitionnées de la phase gazeuse dans chaque zone. Le catalyseur qui circule très rapidement entre les deux zones inférieure et supérieure subit donc d'incessants cycles désactivation-régénération très courts. Il est donc possible d'ajuster les paramètres opératoires de fonctionnement du lit fluidisé pour optimiser le processus de déshydratation/régénération. Notamment, le temps de contact entre particules et gaz sera contrôlé par la hauteur de lit fluidisé dans le réacteur. La productivité élevée en acroléine du catalyseur est maintenue en ajustant la pression partielle d'oxygène dans le gaz de fluidisation afin que sa consommation soit totale juste au niveau de l'injection du glycérol ou de la glycérine. Ainsi, ni le glycérol ou la glycérine, ni l'acroléine formée, ne sont en présence d'oxygène et ne peuvent se dégrader par oxydation comme dans le cas d'une co-alimentation inerte/oxygène en lit fixe qui génère des effets néfastes sur la sélectivité. Du point de vue thermique, le fonctionnement est également très favorable puisque les calories générées par la combustion du coke servent à la réaction de déshydratation du glycérol légèrement endothermique. En outre le procédé décrit dans la présente invention bénéficie des avantages du lit fluidisé à savoir : un très bon contact gaz-solide favorisant les échanges de matière et d'énergie entre les deux phases ; une excellente homogénéité de température et de concentration dans l'ensemble du volume réactionnel et source d'une faible perte de charge. D'autre part, dans un lit fluidisé, le soutirage de catalyseur irréversiblement désactivé et l'ajout de catalyseur neuf peut se faire sans arrêter la production.

L'invention concerne aussi un procédé de fabrication de l'aldéhyde-3-(méthylthio)propionique MMP, du 2-hydroxy-4-méthylthiobutyronitrile HMTBN, de la méthionine, de l'acide 2-hydroxy-4-méthylthiobutanoïque HMTBA, des esters de ce derniers, ou du 2-oxo-4-méthylthiobutanoïque KMB, à partir d'acroléine, procédé dans lequel l'acroléine est obtenue par déshydratation catalytique telle que décrite ci-dessus.

L'invention a encore pour objet l'utilisation pour l'obtention en continu d'acroléine par déshydratation catalytique du glycérol ou de glycérine, en présence d'un catalyseur acide, d'un réacteur à lit fluidisé, ledit réacteur répondant aux caractéristiques décrites précédemment pour mettre en oeuvre le procédé de l'invention.

La présente invention est maintenant exposée plus en détails et illustrée à l'appui des figures 1 à 4 et exemples 1, 2, 3 et 4 suivants :
La Figure 1 représente le schéma d'un réacteur à lit fluidisé, selon une variante de l'invention et tel qu'utilisé dans l'exemple 2.
La Figure 2 est un diagramme représentant la conversion du glycérol et la sélectivité en acroléine en fonction du temps de réaction, observés sur le catalyseur 20 wt.%H₄SiW₁₂O₄₀/Q-10 travaillant en lit fixe, et faisant l'objet de l'exemple 1 qui illustre les inconvénients de l'art antérieur.
La Figure 3 est un diagramme représentant la conversion du glycérol et la sélectivité en acroléine en fonction du temps de réaction avec régénération périodique alternée sur le catalyseur 20 wt.%H₄SiW₁₂₀O₄₀/Q-10 travaillant en lit fixe, et faisant l'objet de l'exemple 1.

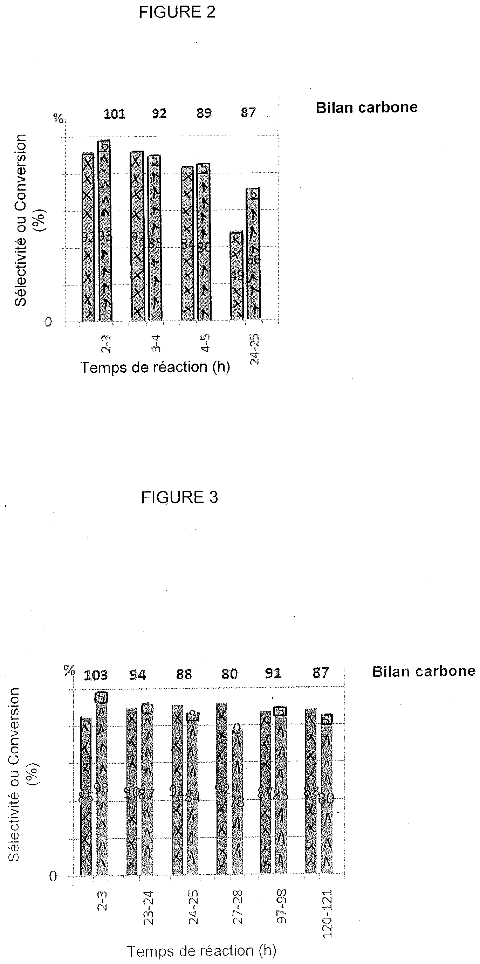

Sur les Figures 2 et 3 :
représente la conversion du glycérol (premiers bâtons)
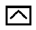 représente la sélectivité en acroléine (seconds bâtons)
□ représente la sélectivité en acétol (bâtons au-dessus des seconds bâtons)

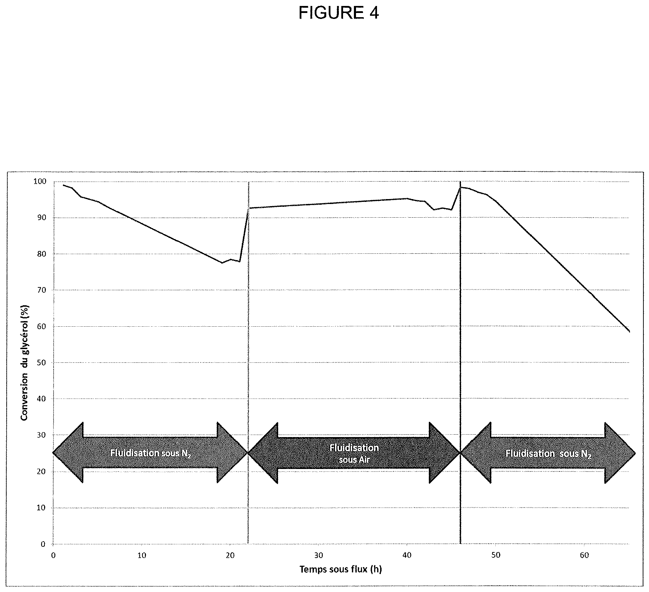

La Figure 4 est un diagramme représentant l'évolution au cours du temps de la conversion du glycérol dans le même réacteur, en fonction du gaz de fluidisation utilisé.

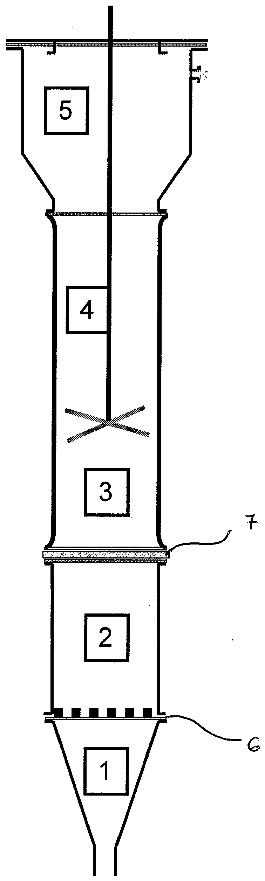

Un exemple de réacteur pouvant être utilisé pour conduire la présente invention est présenté en Figure 1. Il est constitué (de bas en haut) d'une base conique 1 servant de diffuseur de gaz, d'un premier distributeur poreux 6 du gaz de fluidisation; d'une partie cylindrique 2 contenant un solide fluidisable ou non, entourée d'un système de chauffage du gaz, pouvant atteindre une température allant jusque 800 °C ; d'un second distributeur de gaz poreux 7 ; d'un réacteur cylindrique 3 comprenant des prises de pression et de température, non représentées, pouvant aussi servir de système de prélèvement de gaz ; d'une partie 5 de désengagement du solide permettant d'éviter l'entraînement du catalyseur suivie d'un cyclone servant à récupérer les éventuelles particules fines formées ; d'un système 4 d'injection du mélange réactionnel. Ce système 4 coulisse verticalement de manière à pouvoir régler la hauteur d'injection dans le lit fluidisé et ainsi créer les deux zones au sein du lit fluidisé (zone de catalyse acide et zone de décokage du catalyseur) à l'endroit désiré. La solution de glycérol ou de glycérine est injectée de préférence après avoir été préalablement vaporisée. Le système d'injection a été conçu de manière à contrôler le régime d'écoulement du fluide le traversant et ainsi à optimiser le contact gaz-solide à son extrémité.

### Exemple 1 (comparatif) : déshydratation catalytique du glycérol en acroléine selon un procédé de l'art antérieur

La réaction est tout d'abord menée en réacteur à lit fixe avec un catalyseur hétéropolyacide (diamètre des particules 221 µm, surface spécifique = 218 m²/g) déposé sur silice.

Le catalyseur est synthétisé de la manière suivante :
Un réacteur de type autoclave est rempli avec 20 mL d'eau distillée et 1,6 g de support silice de type Cariact Q10 (FUJI SILYSIA CHEMICAL). Après avoir fermé le réacteur, le système d'agitation est mis en route et la température est fixée à 45 °C. Une solution de 0,4 g de H₄SiW₁₂O₄₀ dissous dans 5 mL d'eau distillée est ajoutée au réacteur à l'aide d'un tube plongeant à une vitesse de 1 mL/min. Après addition, le mélange est agité pendant 2 h supplémentaires avant d'être transféré dans un ballon afin d'évaporer le solvant à 70 °C sous vide à l'aide d'un évaporateur rotatif. La poudre obtenue est séchée à l'étuve à 70 °C pendant 8 h.

La réaction catalytique d'essai est effectuée à 250 °C avec 300 mg de catalyseur placés dans un réacteur à lit fixe en acier inoxydable (15 mm de diamètre intérieur, longueur de 120 mm). Le flux de réaction est composé d'une solution aqueuse de glycérol à 10 % en poids alimentée par une pompe HPLC à raison de 1,5 mL/h. La solution est évaporée à 210 °C et diluée dans de l'hélium (30 mL/min) avant d'être introduite dans le réacteur. Les produits de réaction sont condensés dans des pièges froids chaque heure au cours des 5 premières heures sous flux puis une fois de plus après 24 h de réaction. La durée de récupération des produits obtenus dans le piège est d'une heure. Les produits condensés sont analysés par HPLC. Les résultats sont présentés sur la Figure 2.

On constate une diminution importante des performances catalytiques avec le temps de réaction. Ainsi, après 24 h sous flux, les sélectivités en acroléine et la conversion du glycérol sont toutes deux fortement réduites, 46 % et 29 % respectivement, en raison du cokage important à la surface du catalyseur.

Dans un second temps, la réaction est menée en alternant une alimentation constituée d'une solution aqueuse de glycérol à 10 % en poids et une alimentation constituée d'air (7 NmL/min). Le ratio entre les durées de chaque phase est de 1. Les résultats obtenus sont présentés dans la Figure 3.

Une régénération rapide permet donc au catalyseur de conserver d'excellentes performances, 89 % de conversion en moyenne et 85 % de sélectivité en moyenne, sur une longue période (120 h dans cet exemple).

### Exemple 2 : déshydratation catalytique du glycérol en acroléine selon l'invention

Dans l'une des réalisations possibles de la présente invention, les dimensions du réacteur sont les suivantes
- La partie conique (1) du réacteur présente une entrée basse pour le gaz de 6,32 mm de diamètre et de 20 mm de long. La hauteur totale de la partie conique 1 est de 85 mm avec un diamètre interne dans sa partie la plus large de 50 mm et dans sa partie la plus étroite de 6,32 mm ;
- La partie cylindrique 2 servant à chauffer le gaz mesure 255 mm de hauteur et présente un diamètre interne de 50 mm ;
- De la même façon, le corps du réacteur 3 présente un diamètre interne de 50 mm avec une hauteur de 700 mm ;
- Enfin, la partie de désengagement 5 mesure 270 mm de hauteur, possède un diamètre interne de 50 mm pour la partie basse et de 90 mm pour la partie haute.

Les produits de réaction sont récupérés dans un piège froid et sont ensuite analysés sur HPLC. Les composés non condensables sont analysés en ligne par spectrométrie de masse et chromatographie en phase gazeuse.

### Exemple 3 : déshydratation catalytique du glycérol en acroléine selon l'invention

Dans le réacteur de l'exemple 2 sont placés 5 g du catalyseur de l'exemple 1 (diamètre des particules 221 µm, surface spécifique= 218 m²/g) mélangés à 76 g de support de silice de type Cariact Q10 (Fuji Silysia Chemical) de 293 µm de diamètre. L'ensemble est fluidisé, soit par un flux de diazote, soit par un flux d'air, injecté à raison de 90 L/h (275°C, Pₐₜₘ).

0,04 mL/min d'une solution aqueuse de glycérol à 20% en masse diluée par 5,5 L/h (275°C, Pₐₜₘ) de diazote sont injectés à 3 cm au-dessus du distributeur du gaz de fluidisation. Le lit fluidisé est maintenu à 275°C et à pression atmosphérique.

Dans la première partie de l'expérience le diazote est utilisé comme gaz de fluidisation. La Figure 4 montre une diminution progressive de la conversion du glycérol de 98% initialement à 78% après 21 h sous flux réactionnel. Ce phénomène de désactivation est lié à la formation de coke à la surface du catalyseur empêchant ainsi l'accès du glycérol aux sites acides actifs. Dans un second temps, tout autre paramètre restant égal par ailleurs, le diazote est substitué par l'air en tant que gaz de fluidisation. Les résultats montrent que la conversion en glycérol remonte immédiatement à 92% et reste stable à cette valeur très élevée pendant les 25 h suivantes. Lorsqu'on rebascule à nouveau sous flux de diazote, la chute de la conversion n'est pas immédiate ce qui atteste d'une régénération effective du catalyseur par combustion du coke formé à la surface du catalyseur. Evidemment, en l'absence d'oxygène dans le lit fluidisé, le phénomène de cokage conduit à nouveau à une désactivation progressive du catalyseur comme observé précédemment. Les cycles de cokage/régénération peuvent être reproduits sans altération des performances du catalyseur.

### Exemple 4 : déshydratation catalytique du glycérol en acroléine selon l'invention

Dans le réacteur de l'exemple 2 sont placés 5 g du catalyseur de l'exemple 1 (diamètre des particules 221 µm, surface spécifique= 218 m²/g) mélangés à 76 g de support de silice de type Cariact Q10 (Fuji Silysia Chemical) de 293 µm de diamètre. L'ensemble est fluidisé par un flux de diazote injecté à raison de 90 L/h (275°C, Pₐₜₘ). 0,04 mL/min d'une solution aqueuse de glycérol à 20% en masse diluée par 5,5 L/h (275°C, Patm) de diazote est injectée pendant 2 h à 3 cm au-dessus du distributeur de gaz de fluidisation. Le lit fluidisé est maintenu à 275°C et à pression atmosphérique. Ce prétraitement assure la formation d'une couche de coke à la surface du catalyseur. Après arrêt de l'alimentation en glycérol une période de purge par le diazote est respectée afin d'évacuer les éventuelles traces de glycérol restant dans le réacteur. Le diazote est alors substitué par l'air en tant que gaz de fluidisation. La température est mesurée à l'aide de trois thermocouples placés dans la partie basse, au milieu et dans la partie haute du réacteur à lit fluidisé. Avant le passage sous air les 3 températures sont égales à 275°C±2°C. Dès l'injection de l'air on note une évolution du profil de température dans le lit fluidisé. A leur maxima les températures relevées dans la partie basse, au milieu et dans la partie haute du lit fluidisé sont de 293°C, 278°C et 279°C respectivement. Ces relevés attestent que la combustion du coke, très exothermique, s'opère dans la partie basse du lit fluidisé. Après quelques minutes les trois températures redeviennent égales à la valeur nominale (i.e. 275°C) attestant de la bonne homogénéité du lit fluidisé et de la combustion rapide du coke. Cette combustion est également confirmée par un pic de CO₂ détecté dans l'effluent du réacteur lors de cette expérience (analyse réalisée par spectrométrie de masse).

## Revendications

1. Procédé continu d'obtention d'acroléine par déshydratation catalytique de glycérol ou de glycérine, en présence d'un catalyseur acide, **caractérisé en ce qu'**il comprend la régénération concomitante dudit catalyseur et **en ce qu'**il est réalisé dans un réacteur à lit fluidisé, ledit réacteur comprenant deux zones, une première zone, ou zone inférieure, dite de régénération du catalyseur, dans laquelle un gaz de fluidisation comprenant de l'oxygène est introduit et une seconde zone, ou zone supérieure, dite de réaction, dans laquelle le glycérol ou la glycérine est introduit et converti en acroléine, ladite zone de réaction étant dépourvue d'oxygène.

2. Procédé selon la revendication 1, **caractérisé en ce que** le glycérol ou la glycérine est introduit sous la forme d'une solution aqueuse en une concentration variant de 10 à 90% en poids.

3. Procédé selon la revendication 2, **caractérisé en ce que** la solution de glycérol ou de glycérine est introduite sous forme vaporisée.

4. Procédé selon l'une quelconque des revendications 1 à 3, **caractérisé en ce que** le catalyseur acide est choisi parmi les zéolithes, les phosphates, les hétéropolyacides, éventuellement supportés et/ou dopés, les catalyseurs de types oxydes, oxydes supportés, ou encore de type zircones modifiées et/ou dopées.

5. Procédé selon la revendication 4, **caractérisé en ce que** le catalyseur est choisi parmi les hétéropolyacides supportés ou non, dopés par au moins un métal choisi parmi Cs, Rb, Ca, Fe, Zr, La, Hf et Bi.

6. Procédé selon l'une quelconque des revendications 1 à 5, **caractérisé en ce que** la température de déshydratation varie de 180 à 500°C.

7. Procédé selon l'une quelconque des revendications 1 à 6, **caractérisé en ce que** le gaz de fluidisation est choisi parmi l'air, O₂ et un mélange O₂-N₂ contenant jusqu'à 21 % molaire de dioxygène.

8. Procédé selon l'une quelconque des revendications 1 à 7, **caractérisé en ce que** le gaz de fluidisation est chauffé à une température variant de 180 à 800°C.

9. Procédé selon la revendication 8, **caractérise en ce que** la pression varie de la pression atmosphérique à 10 bars.

10. Procédé selon l'une quelconque des revendications 1 à 9, **caractérisé en ce que** la zone supérieure du réacteur comprend, de bas en haut, i) une partie d'introduction du glycérol ou de la glycérine, ii) une partie de réaction par déshydratation catalytique du glycérol, et iii) une partie de désengagement des particules solides fines formées.

11. Procédé selon l'une quelconque des revendications 1 à 10, **caractérisé en ce que** la zone inférieure du réacteur comprend une partie conique d'introduction du gaz de fluidisation.

12. Procédé de fabrication de l'aldéhyde-3-(méthylthio)propionique MMP, du 2-hydroxy-4-méthylthiobutyronitrile HMTBN, de la méthionine, de l'acide 2-hydroxy-4-méthylthiobutanoïque HMTBA, des esters de ce derniers, ou du 2-oxo-4-méthylthiobutanoïque KMB, à partir d'acroléine, **caractérisé en ce qu'**il met en oeuvre un procédé selon l'une quelconque des revendications 1 à 11.

13. Utilisation pour l'obtention en continu d'acroléine par déshydratation catalytique de glycérol ou de glycérine, en présence d'un catalyseur acide, d'un réacteur à lit fluidisé, ledit réacteur comprenant deux zones, une première zone, ou zone inférieure, dite de régénération du catalyseur dans lequel un gaz de fluidisation comprenant de l'oxygène est introduit et une seconde zone, ou zone supérieure, dite de réaction dans lequel le glycérol est introduit, ladite zone de réaction étant dépourvue d'oxygène.

## Patentansprüche

1. Kontinuierliches Verfahren zum Erhalt von Acrolein durch katalytische Dehydratierung von Glycerol oder Glycerin in Anwesenheit eines sauren Katalysators, **dadurch gekennzeichnet, dass** es die gleichzeitige Regeneration des Katalysators umfasst, und dadurch, dass es in einem Reaktor mit Wirbelbett durchgeführt wird, wobei der Reaktor zwei Zonen umfasst, eine erste Zone oder untere Zone, genannt zur Regeneration des Katalysators, in die ein Wirbelschichtgas, umfassend Sauerstoff, eingeführt wird, und eine zweite Zone oder obere Zone, genannt zur Reaktion, in die das Glycerol oder das Glycerin eingeführt und in Acrolein umgewandelt wird, wobei die Reaktionszone frei von Sauerstoff ist.

2. Verfahren nach Anspruch 1, **dadurch gekennzeichnet, dass** das Glycerol oder das Glycerin in Form einer wässrigen Lösung in einer Konzentration eingeführt wird, die von 10 bis 90 Gew% variiert.

3. Verfahren nach Anspruch 2, **dadurch gekennzeichnet, dass** die Lösung aus Glycerol oder aus Glycerin in einer verdampften Form eingeführt wird.

4. Verfahren nach einem der Ansprüche 1 bis 3, **dadurch gekennzeichnet, dass** der saure Katalysator ausgewählt ist aus den Zeolithen, Phosphaten, Heteropolysäuren, eventuell getragen und/oder dotiert, Katalysatoren vom Typ Oxide, getragene Oxide oder auch vom Typ modifizierte und/oder dotierte Zirkone.

5. Verfahren nach Anspruch 4, **dadurch gekennzeichnet, dass** der Katalysator ausgewählt ist aus den getragenen oder nicht getragenen Heteropolysäuren, dotiert durch mindestens ein Metall, ausgewählt aus Cs, Rb, Ca, Fe, Zr, La, Hf und Bi.

6. Verfahren nach einem der Ansprüche 1 bis 5, **dadurch gekennzeichnet, dass** die Dehydratierungstemperatur von 180 bis 500 °C variiert.

7. Verfahren nach einem der Ansprüche 1 bis 6, **dadurch gekennzeichnet, dass** das Wirbelschichtgas ausgewählt ist aus Luft, O₂ und eine Mischung O₂-N₂, enthaltend bis zu 21 Mol% Disauerstoff.

8. Verfahren nach einem der Ansprüche 1 bis 7, **dadurch gekennzeichnet, dass** das Wirbelschichtgas auf eine Temperatur erhitzt wird, die von 180 bis 800 °C variiert.

9. Verfahren nach Anspruch 8, **dadurch gekennzeichnet, dass** der Druck vom atmosphärischen Druck bis 10 bar variiert.

10. Verfahren nach einem der Ansprüche 1 bis 9, **dadurch gekennzeichnet, dass** die obere Zone des Reaktors, von unten nach oben, i) einen Teil zur Einführung des Glycerols oder des Glycerins, ii) einen Teil zur Reaktion durch katalytische Dehydratierung des Glycerols, und iii) einen Teil zur Lösung des Eingriffs der gebildeten feinen festen Partikel umfasst.

11. Verfahren nach einem der Ansprüche 1 bis 10, **dadurch gekennzeichnet, dass** die untere Zone des Reaktors einen konischen Teil zur Einführung des Wirbelschichtgases umfasst.

12. Verfahren zur Herstellung von 3-(Methylthio)propionaldehyd MMP, 2-Hydroxy-4-methylthiobutyronitril HMTBN, Methionin, 2-Hydroxy-4-methylthiobutanolsäure HMTBA, Estern dieser Letzteren oder 2-Oxo-4-methylthiobutansäure KMB auf der Grundlage von Acrolein, **dadurch gekennzeichnet, dass** es ein Verfahren nach einem der Ansprüche 1 bis 11 ausführt.

13. Verwendung zum kontinuierlichen Erhalt von Acrolein durch katalytische Dehydratierung von Glycerol oder Glycerin in Anwesenheit eines sauren Katalysators, eines Reaktors mit Wirbelbett, wobei der Reaktor zwei Zonen umfasst, eine erste Zone oder untere Zone, genannt zur Regeneration des Katalysators, in den ein Wirbelschichtgas, umfassend Sauerstoff, eingeführt wird, und eine zweite Zone oder obere Zone, genannt zur Reaktion, in den das Glycerol eingeführt wird, wobei die Reaktionszone frei von Sauerstoff ist.

## Claims

1. A continuous process for obtaining acrolein by catalytic dehydration of glycerol or glycerin, in the presence of an acid catalyst, **characterized in that** it comprises the concomitant regeneration of said catalyst and **in that** it is carried out in a fluidized bed reactor, said reactor comprising two areas, a first area, or lower area, called catalyst regeneration area, in which a fluidizing gas comprising oxygen is introduced and a second area, or upper area, called reaction area, in which the glycerol or the glycerin is introduced and converted into acrolein, said reaction area being oxygen-free.

2. The process according to claim 1, **characterized in that** the glycerol or the glycerin is introduced in the form of an aqueous solution at a concentration varying from 10 to 90 % by weight.

3. The process according to claim 2, **characterized in that** the glycerol or glycerin solution is introduced in vaporized form.

4. The process according to any one of claims 1 to 3, **characterized in that** the acid catalyst is selected from zeolites, phosphates, heteropolyacids, possibly supported and/or doped, the catalysts of the types oxides, supported oxides, or of the type modified and/or doped zirconia.

5. The process according to claim 4, **characterized in that** the catalyst is selected from heteropolyacids, supported or not, doped by at least one metal selected from Cs, Rb, Ca, Fe, Zr, La, Hf and Bi.

6. The process according to any one of claims 1 to 5, **characterized in that** the dehydration temperature ranges from 180 to 500°C.

7. The process according to any one of claims 1 to 6, **characterized in that** the fluidizing gas is selected from air, O₂ and an O₂-N₂ mixture containing up to 21 mole % of dioxygen.

8. The process according to any one of claims 1 to 7, **characterized in that** the fluidizing gas is heated to a temperature ranging from 180 to 800°C.

9. The process according to claim 8, **characterized in that** the pressure ranges from the atmospheric pressure to 10 bars.

10. The process according to any one of claims 1 to 9, **characterized in that** the upper area of the reactor comprises, from bottom to top, i) a portion for introducing the glycerol or the glycerin, ii) a portion for reacting the glycerol by catalytic dehydration, and iii) a portion for disengaging the formed fine solid particles.

11. The process according to any one of claims 1 to 10, **characterized in that** the lower area of the reactor comprises a conical portion for introducing the fluidizing gas.

12. A method for producing aldehyde-3-(methylthio)propionic MMP, 2-hydroxy-4-methylthiobutyronitrile HMTBN, methionine, 2-hydroxy-4-methylthiobutanoic acid HMTBA, esters of the latter, or 2-oxo-4-methylthiobutanoic KMB, from acrolein, **characterized in that** it implements a process according to any one of claims 1 to 11.

13. A use of a fluidized bed reactor for continuously obtaining acrolein by catalytic dehydration of glycerol or glycerin, in the presence of an acid catalyst, said reactor comprising two areas, a first area, or lower area, called catalyst regeneration area in which a fluidizing gas comprising oxygen is introduced and a second area, or upper area, called reaction area in which the glycerol is introduced, said reaction area being oxygen-free.
